# EUROPÄISCHE PATENTSCHRIFT

(11) **EP 3 979 890 B1**
(45) Veröffentlichungstag und Bekanntmachung des Hinweises auf die Patenterteilung: **03.01.2024**
(21) Anmeldenummer: 21739272.9
(22) Anmeldetag: 22.06.2021
(51) Int. Cl.: A61B 90/50

(54) **MOTORISIERTER POSITIONIERARM**
MOTORIZED POSITIONING ARM
BRAS DE POSITIONNEMENT MOTORISÉ

(30) Priorität: 26.08.2020 DE 102020122352
(43) Veröffentlichungstag der Anmeldung: 13.04.2022
(73) Patentinhaber: Isys Medizintechnik GmbH, 6370 Kitzbühel (AT)
(72) Erfinder: VOGELE, Michael, 86830 Schwabmünchen (DE)
(74) Vertreter: Keller Schneider Patentanwaltsgesellschaft mbH
(86) Internationale Anmeldenummer: PCT/EP2021/067028
(87) Internationale Veröffentlichungsnummer: WO 2022/042898

(56) Entgegenhaltungen:
- EP-A1- 2 379 284
- WO-A1-2017/144172
- DE-A1-102014 016 824
- GB-A- 2 515 476
- US-A1- 2018 264 655

## Beschreibung

### Technisches Gebiet

Die vorliegende Erfindung betrifft einen motorisierten Positionierarm zum Positionieren von Instrumenten, insbesondere medizintechnischen Instrumenten.

### Stand der Technik

Aus dem Stand der Technik sind für unterschiedliche Anwendungen verschiedenste Positionierarme mit unterschiedlichsten Anforderungen bekannt. Insbesondere bei Positionierarmen zum Positionieren von medizintechnischen Instrumenten werden beispielsweise sehr hohe Anforderung an die Stabilität, Zuverlässigkeit und Bedienbarkeit der Positionierarme gestellt. So sind im Stand der Technik zahlreiche Versuche unternommen worden, Positioniersysteme unter Berücksichtigung dieser Anforderungen weiterzuentwickeln und zu verbessern.

Beispielsweise ist aus der Druckschrift WO 2017 / 144 172 A1 ein Positionierarm mit einem Drehkörper bzw. Drehgelenk und zwei daran angelenkten Armelementen bekannt. Dabei ist der Winkel zwischen den beiden Armelementen durch eine an dem Drehkörper über eine I<upplung angebrachte Ratsche feststellbar, wobei an der I<upplung ein Schalter vorgesehen ist, der die Winl<elposition der Arme in einer Schalterstellung zueinander verriegelt.

Der aus der Druckschrift WO 2017 / 144 172 A1 bekannte Positionierarm hat den Vorteil, dass mittels der Ratsche einfach und mit verhältnismäßig geringem Kraftaufwand ein Feststellen d.h. Blockieren des Drehgelenks des Positionierarms möglich ist. Insbesondere ist die Ratsche auch in einem sterilen Umfeld, beispielsweise unter einer sterilen Abdeckfolie, gut bedienbar. Außerdem kann mittels der Ratsche ein bestimmtes Mindest-Drehmoment vorgegeben werden, so dass das Blockieren des Positionierarms zuverlässig erfolgen kann.

Allerdings hat der aus der WO 2017 / 144 172 A1 bekannte Positionierarm immer noch den Nachteil, dass die Bedienung nach wie vor benutzerabhängig ist. So bleibt ein Restrisiko bei der Anwendung, wenn der Anwender die Ratsche nicht richtig betätigt und sich der Arm während eines operativen Eingriffs verstellt. Außerdem ist die Ergonomie bzw. Gebrauchstauglichkeit nach wie vor nicht optimal. So muss der Anwender beispielsweise beim Positionieren des Positionierarms gleichzeitig das Instrument am Ende des Positionierarms halten und das Operationsfeld im Auge behalten. Solch ein simultaner Ablauf ist für den Anwender sehr schwierig, insbesondere, da beim Feststellen des Arms relativ hohe Kräfte aufgewendet werden müssen und die Rotationsbewegung beim Drehen der Ratsche mit Bewegungen der anderen Hand konträr laufen kann. Da der Positionierarm vor, während und nach dem Eingriff oft umpositioniert werden muss, d.h. vielfaches Öffnen und Schließen erforderlich ist, wäre zudem eine schnellere Bedienmöglichkeit wünschenswert. Darüber hinaus muss bei Verwendung einer sterilen Abdeckfolie die Ratsche durch die Folie hindurch unter der sterilen Folie bewegt werden, was einerseits unkomfortabel und andererseits zum Verschleiß oder Versagen der Folie führen kann.

GB 2 515 476 A beschreibt einen Positionierarm zum Positionieren von medizintechnischen Instrumenten, mit zwei Armelementen, die mittels eines zentralen Gelenks schwenkbar um eine Schwenkachse miteinander verbunden sind, und weitere Gelenke an den entgegengesetzten Enden der beiden Armelemente. Der Positionierarm hat einen Blockiermechanismus zum Blockieren und Freigeben des zentralen Gelenks und der weiteren Gelenke und eine zentrale Welle, die koaxial zu der Schwenkachse angeordnet ist, sowie eine Übertragungseinrichtung von der zentralen Welle zu den weiteren Gelenken.

Ausgehend von dem oben aufgeführten Stand der Technik ist es Aufgabe der vorliegenden Erfindung, einen Positionierarm zur Verfügung zu stellen, der die Probleme und Nachteile der aus dem Stand der Technik bekannten Systeme behebt und entsprechende Vorteile gegenüber diesen hat. Insbesondere ist es Aufgabe der vorliegenden Erfindung, einen Positionierarm anzugeben, der eine ergonomische, komfortable, schnelle und weitgehend benutzerunabhängige Bedienung des Positionierarms auch durch eine sterile Abdeckfolie hindurch erlaubt.

### Darstellung der Erfindung

Diese Aufgabe wird durch den Gegenstand des unabhängigen Anspruchs 1 gelöst. Weitere mögliche Ausgestaltungen der Erfindung sind insbesondere in den abhängigen Ansprüchen angegeben.

Die erfindungsgemäße Lösung liegt darin, einen motorisierten Positionierarm zum Positionieren von Instrumenten, insbesondere medizintechnischen Instrumenten, anzugeben, wobei der motorisierte Positionierarm mindestens zwei Armelemente aufweist, die mittels eines zentralen Gelenks schwenkbar um eine Schwenkachse miteinander verbunden sind, wobei zumindest eines der Armelemente ein weiteres Gelenk an einem dem zentralen Gelenk entgegengesetzten Ende aufweist, wobei der motorisierte Positionierarm einen Blocl<iermechanismus zum Blockieren und Freigeben des zentralen Gelenks und des zumindest einen weiteren Gelenks aufweist, wobei der Blocl<iermechanismus eine zentrale Welle, die koaxial zu der Schwenkachse angeordnet ist, und zumindest eine Übertragungseinrichtung von der zentralen Welle zu dem zumindest einen weiteren Gelenk aufweist, wobei der Blocl<iermechanismus eine elektrische Blocl<iereinrichtung aufweist, die dazu ausgebildet ist, mit der zentralen Welle in Eingriff zu stehen, um das Blockieren und Freigeben der Gelenke zu ermöglichen, und wobei vorzugsweise die elektrische Blocl<iereinrichtung dazu ausgebildet ist, sich entlang der zentralen Welle zu bewegen.

Der motorisierte Positionierarm dient insbesondere dazu, während eines Eingriffs an einem Patienten medizinische Instrumente zuverlässig d.h. sicher und präzise zu positionieren bzw. zu halten. Insbesondere bei chirurgischen oder bioptischen Eingriffen ist ein solch zuverlässiges Positionieren der Instrumente, beispielsweise chirurgischer Haken, Endoskope oder Nadeln, unerlässlich. Der motorisierte Positionierarm erlaubt ein motorisiertes Blockieren der Gelenke. Die Bewegungen bzw. das Positionieren des Positionierarms selbst kann durchaus manuell erfolgen.

Bei der Verwendung des motorisierten Positionierarms zum Positionieren medizinischer Instrumente kann beispielsweise eines der Armelemente über ein weiteres Gelenk mit einer Befestigungseinrichtung für einen OP-Tisch verbunden ausgebildet sein. Das andere Armelement kann entsprechend über ein weiteres Gelenk mit einem Instrumentenhalter verbunden ausgebildet sein. Der Instrumentenhalter ist vorzugsweise als Klemmverbindung ausgebildet. So kann ein medizintechnischen Instrument von außen in den Instrumentenhalter geklemmt werden, ohne eine sterile Abdeckfolie zu verletzen, die den motorisierten Positionierarm einhüllt.

Die zentrale Welle, die koaxial zu der Schwenkachse der beiden Armelemente ausgebildet ist, kann ein Gewinde aufweisen. Beispielsweise kann es sich bei der zentralen Welle um einen Spannbolzen handeln.

Die Übertragungseinrichtung kann beispielsweise mindestens eine Spannhülse aufweisen, die auf der zentralen Welle angeordnet ist. Die Spannhülse kann als Gewindehülse ausgebildet sein, die mit dem Gewinde der zentralen Welle in Eingriff steht, oder axial verschiebbar auf der zentralen Welle angeordnet sein.

Die Übertragungseinrichtung kann ferner eine Schubstange aufweisen, die axial innerhalb des Armelements verläuft und derart mit der Spannhülse in Wirkverbindung steht, dass die Übertragungseinrichtung das weitere Gelenk am Ende des Armelements sperren d.h. blockieren kann. Hierzu kann die Spannhülse beispielsweise eine geneigte Abrollfläche aufweisen, an der ein Übertragungskörper, beispielsweise eine I<ugel, anliegt. Alternativ hierzu kann die Spannhülse beispielsweise eine Gelenkpfanne aufweisen, in der ein an dem Übertragungskörper angeordneter Nocken eingesetzt ist. Insgesamt kann mittels der Übertragungseinrichtung eine Axialbewegung der zentralen Welle in eine Axialbewegung der Schubstange übertragen werden. Dabei führt die Axialbewegung der Schubstange zu einem Blockieren des entsprechenden Gelenks am Ende des Armelements.

Obwohl das Blockieren mittels der Blocl<iereinrichtung primär elektrisch erfolgt, kann zusätzlich ein manuelles Blockieren bzw. Freigeben der Gelenke möglich sein. So kann die Blocl<iereinrichtung auch ohne elektrische Energie betrieben werden. Die zum Betrieb der Blocl<iereinrichtung benötigte elektrische Energie wird vorzugsweise mittels eines Akkumulators geliefert. So ist keine Verkabelung nötig, die innerhalb des OP-Raums eine Gefahrenquelle darstellen kann.

Mit dem erfindungsgemäßen motorisierten Positionierarm wird die Aufgabe in zufriedenstellender Weise gelöst. Als motorisierte Antriebseinheit bzw. Aktuator ist die elektrische Blocl<iereinrichtung sehr komfortabel und einfach bedienbar, insbesondere auch durch eine sterile Abdeckfolie hindurch. Wenn eine motorisierte Antriebseinheit verwendet wird, müssen keine Elemente, wie beispielsweise die Ratsche, unter der sterilen Abdeckfolie bewegt werden. Neben der Vereinfachung der Bedienung ist so auch der Verschleiß der sterilen Abdeckfolie geringer. Außerdem ist die Bedienung zumindest im Wesentlichen benutzerunabhängig ausgebildet. Im Gegensatz zu einer manuellen Blocl<iereinrichtung kann der Blocl<iermechanismus hier nicht zu schwach eingestellt werden, so dass der motorisierte Positionierarm stets stabil genug ist und sich beim Eingriff nicht verstellen kann. Ferner erlaubt die motorisierte Blocl<iereinrichtung eine sehr schnelle Bedienung. Ein vollständiges Blockieren der Gelenke des motorisierten Positionierarms kann innerhalb weniger Sekunden erreicht werden.

Ein weiterer Vorteil der elektrischen d.h. elektrisch betriebenen Blocl<iereinrichtung liegt in ihrer Kompaktheit. Bei elektrischen Antrieben gab es in den vergangenen Jahren enorme technische Weiterentwicklungen. Alternative Drucksysteme (durch Druckluft, Öl etc.) sind zu kostenintensiv in der Herstellung, komplex in der Anwendung und Wartung, und stellen aufgrund der verwendeten Flüssigkeiten für das sterile Operationsfeld und den Anwender ein vermeidbares Risiko dar. Gerade hinsichtlich der Sterilität ist vorteilhaft, dass elektrische Antriebe d.h. Motoren inklusive der dazugehörigen Elektronik mittlerweile auch sterilisierbar, insbesondere autoklavierbar, verfügbar sind. Dies ist insbesondere bei Anwendungen relevant, bei denen man nicht mit einer sterilen Abdeckfolie arbeiten kann.

Ein weiterer Vorteil des motorisierten Positionierarms liegt darin, dass die Blocl<iereinrichtung an der zentralen Welle ansetzt. Hierdurch wird ein progressives Blockieren der Gelenke ermöglicht. Da die Blocl<iereinrichtung zentral und von außen über die zentrale Welle in den mechanischen Blocl<iermechanismus des motorisierten Positionierarms eingreifen kann, lassen sich die Übertragungseinrichtungen, beispielsweise Schubstangen, im Inneren der Armelemente mit hohen Zug- und Druckkräften simultan oder progressiv bewegen. Durch diese progressive Bewegung ist es dann möglich, den gesamten motorisierten Positionierarm bzw. die einzelnen Gelenke des Arms progressiv zu schließen und zu öffnen. Insbesondere das progressive Öffnen der einzelnen Gelenke kann erwünscht sein, um zu verhindern, dass es nur einen vollständig geöffneten Zustand gibt, in dem sich der motorisierte Positionierarm instabil hin und her bewegt.

Wenn die elektrische Blocl<iereinrichtung dazu ausgebildet ist, sich relativ, insbesondere entlang, der zentralen Welle zu bewegen, ist die elektrische Blocl<iereinrichtung als bewegliche Spanneinheit ausgebildet. Insbesondere ist diese dann auch relativ zu anderen Teilen des Positionierarms, beispielsweise relativ zu dem Armelement, an dem die elektrische Blocl<iereinrichtung angeordnet ist, bewegbar ausgebildet.

Zum Bewegen der elektrischen Blocl<iereinrichtung kann die zentrale Welle beispielsweise ein Außengewinde aufweisen, das mit einem Bauteil der elektrischen Blocl<iereinrichtung in Kontakt bringbar ist, das ein Innengewinde aufweist. Wenn das das Innengewinde aufweisende Bauteil gedreht wird, bewegt sich das Bauteil im Gewindeeingriff an der zentralen Welle auf oder ab. Bei dem Bauteil kann es sich beispielsweise um ein Ausgangszahnrad der elektrischen Blocl<iereinrichtung handeln. Insbesondere ist das Bauteil derart mit dem Rest der Blocl<iereinrichtung verbunden, dass der Rest der Blocl<iereinrichtung sich gemeinsam mit dem Bauteil axial entlang der zentralen Welle bewegt. Hierzu kann das Bauteil beispielsweise an einem Basiskörper der Blocl<iereinrichtung angeordnet sein. Insbesondere kann das Bauteil mittels eines Lagers, beispielsweise eines Trocl<enlagers, an dem Basiskörper angeordnet sein. Das Bauteil kann sich dann relativ zu dem Basiskörper drehen, nimmt den Basiskörper allerdings in axialer Richtung mit. Bei dem Basiskörper kann es sich beispielsweise um ein Winl<elstücl< handeln.

Bei einer bevorzugten Ausgestaltung der vorliegenden Erfindung weisen beide Armelemente jeweils das weitere Gelenk an dem dem zentralen Gelenk entgegengesetzten Ende auf, wobei der Blocl<iermechanismus jeweils eine Übertragungseinrichtung zu dem weiteren Gelenk aufweist.

Dabei können beide Übertragungseinrichtungen wie bereits beschrieben ausgebildet sein. Beispielsweise kann auch eine Spannhülse axial verschiebbar auf der zentralen Welle und eine weitere Spannhülse in Gewindeeingriff mit der zentralen Welle ausgebildet sein. Ferner ist auch eine Tandemlösung denkbar, bei der die eine Spannhülse von der anderen mitgenommen wird. Beispielsweise kann das zentrale Gelenk dadurch blockiert werden, dass Reibflächen der beiden Spannhülsen aneinanderdrücken. Die genaue Ausbildung ist hierbei allerdings nebensächlich.

Eine vorteilhafte Ausführungsform sieht vor, dass das weitere Gelenk als I<ugelgelenl< ausgebildet ist.

Ein I<ugelgelenl< ist ein Gelenk mit einer frei dreh- und schwenkbaren I<ugel. Die Übertragungseinrichtungen können hier beispielsweise in den Armelementen untergebrachte Hülsen aufweisen, die mittels der jeweiligen Schubstange gegen das I<ugelgelenl< drücken und so ein festes Verspannen der Gelenke ermöglichen.

Gemäß einer vorteilhaften Weiterbildung der Erfindung weist die elektrische Blocl<iereinrichtung einen Elektromotor mit einer Ausgangswelle auf, wobei die Ausgangswelle senkrecht zu der zentralen Welle ausgerichtet ist.

Bei einer derartigen Ausbildung liegt der Vorteil in einer besonders hohen Kompaktheit. Die elektrische Blocl<iereinrichtung kann platzsparend an einem der Armelemente angeordnet oder auch in diesen integriert sein. Üblicherweise liegen bei den meisten Anwendungen nur sehr begrenzte Platzverhältnisse vor und ein bestmöglichster Zugang vom Operateur zu dem Operationsfeld ist von hoher Relevanz. Motorisierte Positionierarme, die lateral zu weit ausladend sind, lassen sich oftmals im Operationsfeld nicht gut genug bedienen.

Dennoch wäre alternativ zu der senkrechten Anordnung selbstverständlich auch ein Direktantrieb in Achsrichtung der zentralen Welle denkbar. Eine solche Ausführung hätte den Vorteil, dass die konstruktive Umsetzung der Anbringung der elektrischen Blocl<iereinrichtung technisch leicht umsetzbar ist.

Eine besonders vorteilhafte Ausgestaltung der vorliegenden Erfindung sieht vor, dass die elektrische Blocl<iereinrichtung ein I<egelradgetriebe mit einem Eingangszahnrad, das mit der Ausgangswelle des Elektromotors verbunden ist, und einem Ausgangszahnrad aufweist, das mit der zentralen Welle in Eingriff ist.

Ein Vorteil liegt auch hier in der besonderen Kompaktheit des I<egelradgetriebes. Ferner kann mittels des I<egelradgetriebes ein hoher und übersetzungsunabhängiger Wirkungsgrad erreicht werden.

Gemäß einer vorteilhaften Weiterentwicklung dieser Ausführungsform ist das Ausgangszahnrad mittels zumindest im Wesentlichen ringförmigen Trocl<enlager gelagert.

Die Verwendung von Trocl<enlagern ist hier vorteilhaft, da Flüssigkeiten im Medizinbereich immer als Risikofaktor gelten, und zwar selbst wenn die relevanten Teile insgesamt alle innenliegend und gekapselt im Gehäuse vorliegen. Vorzugsweise sind die Trocl<enlager aus Gleitl<unststoff ausgebildet. Hierdurch wird eine Trocl<enlagerung bei einem niedrigen und definierten Widerstand möglich. Alternativ wäre allerdings auch die Verwendung von Kugel- und Nadellagern denkbar, wobei diese dann vorzugsweise ohne Schmierstoff verwendet werden.

Gemäß einer vorteilhaften Ausführungsform der Erfindung weist das Ausgangszahnrad ein Innengewinde auf und die zentrale Welle ein zu dem Innengewinde passendes Außengewinde auf.

Hierdurch kann einfach eine Kraftübertragung von der elektrischen Blocl<iereinrichtung auf die zentrale Welle erfolgen.

Bei einer besonders bevorzugten Ausführungsform ist die elektrische Blocl<iereinrichtung an einem der beiden Armelemente angeordnet und die Ausgangswelle des Elektromotors erstreckt sich zumindest im Wesentlichen parallel zu der Axialrichtung des Armelements, an dem die elektrische Blocl<iereinrichtung angeordnet ist.

Dies stellt eine besonders platzsparende und kompakte Bauweise dar, so dass die diesbezüglich bereits diskutierten Vorteile erreicht werden können. Beispielsweise kann die gesamte elektrische Blocl<iereinrichtung kleiner als 70mm x 150mm x 50mm ausgebildet sein.

Gemäß einer vorteilhaften Weiterentwicklung dieser Ausführungsform ist die elektrische Blocl<iereinrichtung lösbar an dem Armelement ausgebildet, an dem die elektrische Blocl<iereinrichtung angeordnet ist.

Aufgrund der lösbaren Ausbildung ist die elektrische Blocl<iereinrichtung nachrüstbar. Die elektrische Blocl<iereinrichtung kann also an einen existierenden nicht motorisierten Positionierarm angebracht werden. So kann man eine manuelle Blockiereinrichtung, wie beispielsweise das eingangs beschriebene System, verbessern.

Alternativ hierzu kann die elektrische Blocl<iereinrichtung auch in dem Armelement integriert sein, an dem die elektrische Blocl<iereinrichtung angeordnet ist. Integriert bedeutet hierbei, dass das Armelement und die elektrische Blocl<iereinrichtung ein zumindest bereichsweise gemeinsames Gehäuse aufweisen.

Eine vorteilhafte Ausgestaltung des motorisierten Positionierarms sieht weiterhin vor, dass die elektrische Blocl<iereinrichtung als bewegliche Spanneinheit ausgebildet ist, die relativ zu dem Armelement, an dem die elektrische Blocl<iereinrichtung angeordnet ist, in Axialrichtung der zentralen Welle bewegbar ausgebildet ist.

Die elektrische Blocl<iereinrichtung ist also schwimmend gelagert ausgebildet. Bei einer fixen am Armelement montierten Ausführung nähern sich die Zahnräder je nach Öffnungsgrad des motorisierten Positionierarms entweder an oder entfernen sich voneinander. So ergeben sich insbesondere hinsichtlich Verschleiß der sich bewegenden, starren Teile große Nachteile. Gerade in der Medizin ist Verschleiß ein kritisches Problem. Sämtliche Systeme müssen Sicherheit über die gesamte Lebensdauer bieten. Außerdem führt der Verschleiß dazu, dass in der elektrischen Blocl<iereinrichtung nur eine ungenaue Kräftemessung, beispielsweise über Motorströme, Kraftsensoren oder Abstandssensoren, möglich ist. Diese ungenaue Kräftemessung ist allerdings nicht ausreichend, um zu gewährleisten, dass der motorisierte Positionierarm über die elektrische Blocl<iereinrichtung sicher und benutzerunabhängig geöffnet und geschlossen werden kann.

Die elektrische Blocl<iereinrichtung ist als bewegliche Spanneinheit vorzugsweise ausschließlich mittels der zentralen Welle geführt. Allerdings wäre eine weitere Führung lateral durch ein Gehäuse möglich. Dieses Gehäuse kann auch verhindern, dass sich die elektrische Blocl<iereinrichtung beim Blockieren und Freigeben mitdreht.

Gemäß einer weiteren Ausgestaltung der vorliegenden Erfindung weist der motorisierte Positionierarm ein Gehäuse auf, von dem die elektrische Blocl<iereinrichtung zumindest im Wesentlichen umschließbar ist, wobei das Gehäuse griffförmig ausgebildet ist.

Ein Gehäuse, das beispielsweise Teile des Armelements und der elektrischen Blocl<iereinrichtung umfasst, ist vorteilhaft, um den Anwender und den Patienten zu schützen und eine ausreichende Reinigbarkeit und Sterilisierbarkeit zu gewährleisten.

Da das Gehäuse griffförmig ausgebildet ist, kann das Gehäuse auch als Griff verwendet werden, um das entsprechende Armelement zu halten und manuell zu bewegen. Hierbei ist das Gehäuse ergonomisch ausgebildet, so dass das ganze System mit Handschuhen und ggf. durch eine sterile Abdeckfolie bedienbar ist. Zur besseren Griffigkeit kann das Gehäuse zumindest bereichsweise mit Anti-Rutsch-Material beschichtet sein.

Das Gehäuse kann als separates Gehäuse der elektrischen Blocl<iereinrichtung ausgebildet sein. Das Gehäuse kann auch zumindest bereichsweise integral mit dem entsprechenden Armelement ausgebildet sein. Insbesondere wenn das Gehäuse integral mit dem Armelement ausgebildet ist, kann das Gehäuse aus Leichtmetall, beispielsweise Aluminium gefertigt sein.

Das Gehäuse kann einen Deckel aufweisen, um einen einfachen Zugriff auf die elektrische Blocl<iereinrichtung zu ermöglichen. Dieser Deckel kann beispielsweise aus Kunststoff gefertigt sein. Das Gehäuse erfüllt insgesamt vorzugsweise einen Schutz von IP44 nach DIN EN 60529 (VDE 0470-1):2014-09.

Eine vorteilhafte Ausgestaltung des motorisierten Positionierarms sieht vor, dass die elektrische Blocl<iereinrichtung einen Anschlag aufweist, der mit dem Gehäuse in Anschlag gerät und dadurch ein Mitdrehen der elektrischen Blocl<iereinrichtung beim Blockieren und Freigeben der Gelenke verhindert.

Mittels des Gehäuses wird somit eine weitere Funktion erfüllt. Bei einer möglichen alternativen Ausführung kann das Gehäuse der elektrischen Blocl<iereinrichtung eine noch präzisere Führung bieten. Beispielsweise kann eine Linearführung in Richtung der zentralen Welle ausgebildet sein.

Gemäß einer vorteilhaften Weiterbildung der Erfindung sind an dem Gehäuse redundante Bedienelemente zum Ansteuern des Elektromotors angeordnet.

Bei den Bedienelementen kann es sich beispielsweise jeweils um einen Taster bzw. Knopf handeln, der durch Drücken betätigt wird und nach dem Loslassen vorzugsweise selbsttätig in die Ausgangslage zurückkehren kann. Die Bedienelemente sind derart ausgebildet, dass deren Positionen mittels taktiler Wahrnehmung sicher bestimmt werden können. Vorzugsweise sind die Bedienelemente in einer Tasche angeordnet. Hierdurch kann neben einem erleichterten Finden der Knöpfe ein unbeabsichtigtes Drücken derselben verhindern werden. Insgesamt kann so das ganze System vorteilhaft mit Handschuhen und ggf. durch sterile Abdeckfolien bedienbar sein.

Die Bedienelemente sind redundant ausgebildet, um ein unbeabsichtigtes Ansteuern der Blocl<iereinrichtung zu verhindern. Beispielsweise sind vier Bedienelemente, zwei Bedienelemente zum Blockieren und zwei Bedienelemente zum Freigeben der Gelenke ausgebildet. Die Bedienelemente sind so angeordnet, dass der Anwender das griffartige Gehäuse, also das entsprechende Armelement d.h. auch einen großen Teil des Gewichts des motorisierten Positionierarms halten und die Bedienelemente gleichzeitig drücken kann.

Neben den Bedienelementen können auch weitere Einrichtungen redundant ausgebildet sein. Beispielsweise können Drucl<-, Positionssensoren, Wegsensoren und/oder Motorstromsensoren redundant ausgebildet sein. Diese können eine genaue Steuerung der Kraft, die auf das Ausgangszahnrad ausgeübt wird, oder eine genaue Bestimmung des Zustands der Blocl<iereinrichtung ermöglichen. Auch die Verkabelung ist beispielsweise redundant ausgebildet.

Es ist vorteilhaft, verschiedene Arten von Sensoren zu kombinieren, um zusätzliche Sicherheit zu gewinnen. Ferner können auch mehr als zwei Sensorsysteme eingesetzt werden, um zusätzliche Redundanz zu gewinnen.

Bei einer besonders bevorzugten Ausführungsform ist innerhalb des Gehäuses eine Ladespule zum I<abellosen Laden eines zum Antrieb des Elektromotors geeigneten Akkumulators angeordnet.

Die Ausbildung zum kabellosen Laden eliminiert das von I<abeln ausgehende Risiko im OP-Raum. Ferner kann das Gehäuse besonders gut geschlossen ausgebildet sein, da kein Zugang für ein I<abel vorgesehen sein muss. Das Laden des Akkumulators kann mittels eines Ladestücl<s erfolgen. Dieses Ladestücl< kann an dem Gehäuse angeordnet werden. Beispielsweise können das Ladestücl< und das Gehäuse eine derartig komplementäre Geometrie aufweisen, dass ein mechanisches Schlüssel-Schloss-Prinzip ausgebildet ist. So kann ein zuverlässiges Laden des Akkumulators gewährleistet werden.

Gemäß einer vorteilhaften Ausführungsform der Erfindung ist an dem Gehäuse mindestens ein Indikatorelement angeordnet, mittels dessen ein Ladezustand des Akkumulators und/oder ein Blocl<ierzustand des Blocl<iermechanismus anzeigbar ist.

Das Indikatorelement kann also einen Blocl<ierzustand (beispielsweise vollständig blockiert, vollständig freigegeben, teilweise blockiert, halbweich) des motorisierten Positionierarms bzw. der Blocl<iereinrichtung mittels Ton, Vibration und/oder visueller Indikation angeben. Vorzugsweise erfolgt eine visuelle Indikation beispielweise mittels einer LED. Alternativ oder zusätzlich kann auch der Ladezustand, also die Restladung, und/oder der Lademodus (beispielsweise aktiv) des Akkumulators angezeigt werden. Vorteilhaft wird spätestens bei einer Restladung, die noch 10 Blocl<ierungsvorgänge zulässt, das Vorliegen einer niedrigen Ladung angezeigt.

Sämtliche der vorab beschriebenen Vorteile können bei einem motorisierten Positionierarm zum Positionieren von medizintechnischen Instrumenten besonders gut genutzt werden.

### Kurze Beschreibung der Figuren

Weitere Merkmale, Vorteile und Ausführungsformen der Erfindung ergeben sich aus der nachfolgenden Beschreibung anhand der Figuren. Es zeigen:
- Fig. 1: eine Darstellung eines erfindungsgemäßen motorisierten Positionierarms mit geschlossenem Gehäuse,
- Fig. 2: der in Fig. 1 dargestellte motorisierte Positionierarm ohne Deckelement und mit abgehobener Ladeeinrichtung,
- Fig. 3: eine detailliertere Darstellung des erfindungsgemäßen motorisierten Positionierarms mit einem teilweise ausgeblendeten Gehäuse und einer sichtbaren erfindungsgemäßen elektrischen Blockiereinrichtung,
- Fig. 4: eine detailliertere Darstellung des erfindungsgemäßen motorisierten Positionierarms mit einem teilweise ausgeblendeten Gehäuse und einer von einer zentralen Welle abgehobenen erfindungsgemäßen elektrischen Blockiereinrichtung, und
- Fig. 5: eine vereinfachte Schnittansicht des erfindungsgemäßen motorisierten Positionierarms.

### Wege zur Ausführung der Erfindung

Ähnliche Elemente sind in den Figuren allgemein mit gleichen oder ähnlichen Bezugsziffern versehen.

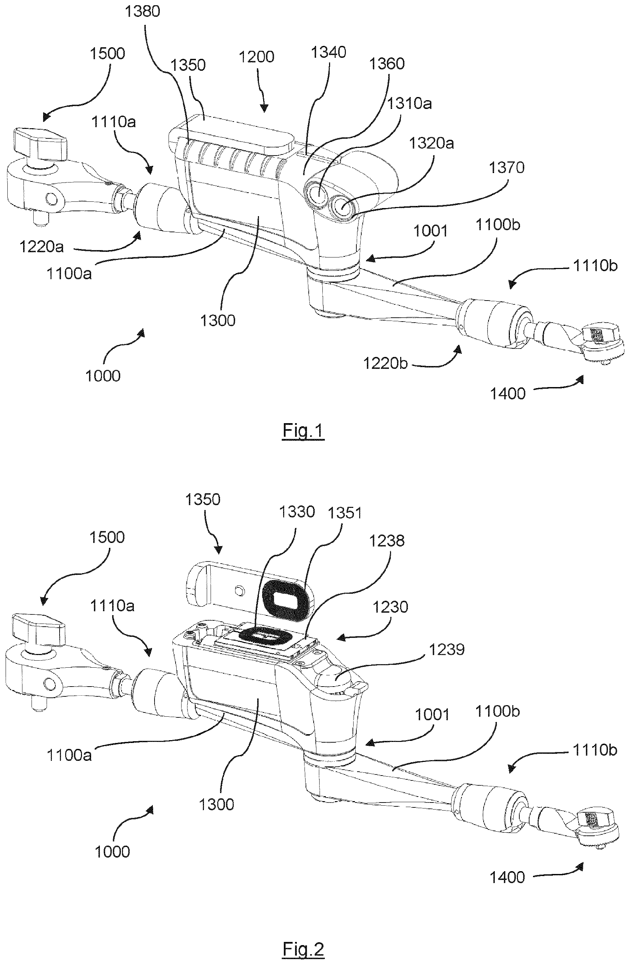

Die Fig. 1 zeigt eine Darstellung eines erfindungsgemäßen motorisierten Positionierarms 1000. Der motorisierte Positionierarm 1000 weist zwei Armelemente 1100a, 1 100b auf. Die beiden Armelemente 1100a und 1100b sind mittels eines zentralen Gelenks 1001 schwenkbar miteinander verbunden.

Das Armelement 1100a weist zwei Enden auf. Ein Ende ist mit dem zentralen Gelenk 1001 verbunden und das andere Ende 1120a ist mit einem weiteren Gelenk 1110a verbunden. Das weitere Gelenk 1110a ist als I<ugelgelenl< ausgebildet. Das weitere Gelenk 1110a ist mit einer Befestigungseinrichtung 1500 verbunden. Hier ist die Befestigungseinrichtung 1500 beispielsweise als schraubbare Verbindung dargestellt. Die Befestigungseinrichtung 1500 kann beispielsweise mit einem OP-Tisch verbunden werden.

Das Armelement 1 100b weist ebenfalls zwei Enden auf. Dabei ist ein Ende mit dem zentralen Gelenk 1001 verbunden und das andere Ende 1120b ist mit einem weiteren Gelenk 1110b verbunden. Das weitere Gelenk 1110b ist ebenfalls als I<ugelgelenl< ausgebildet. Das weitere Gelenk 1110b ist mit einem Instrumentenhalter 1400 verbunden. An dem Instrumentenhalter 1400 kann beispielsweise ein medizintechnisches Instrument angeordnet werden. Auch wenn der Instrumentenhalter 1400 mit einer Schraubverbindung dargestellt ist, ist dieser vorzugsweise als Klemmverbindung ausgebildet. So kann ein passendes Klemmstück an dem medizintechnischen Instrument von außen in den Instrumentenhalter geklemmt werden, ohne eine sterile Abdeckfolie zu verletzen, die den motorisierten Positionierarm 1000 einhüllt.

Alle Gelenke 1001, 1110a, 1110b sind mittels eines gemeinsamen Blocl<iermechanismus 1200 blocl<ierbar und freigebbar. Der Blocl<iermechanismus 1200 ist in Fig. 1 nicht erkennbar, da dieser innerhalb eines Gehäuses 1300 angeordnet ist.

Das Gehäuse 1300 weist ein Deckelelement 1360 auf. Das Deckelelement 1360 kann beispielsweise aus Kunststoff hergestellt sein. Der Rest des Gehäuses 1300 kann, wie in Fig. 1 dargestellt, integral mit dem Armelement 1100a ausgebildet sein. Daher kann der Rest des Gehäuses 1300 aus dem Material des Armelements 1100a, beispielsweise aus Aluminium, ausgebildet sein.

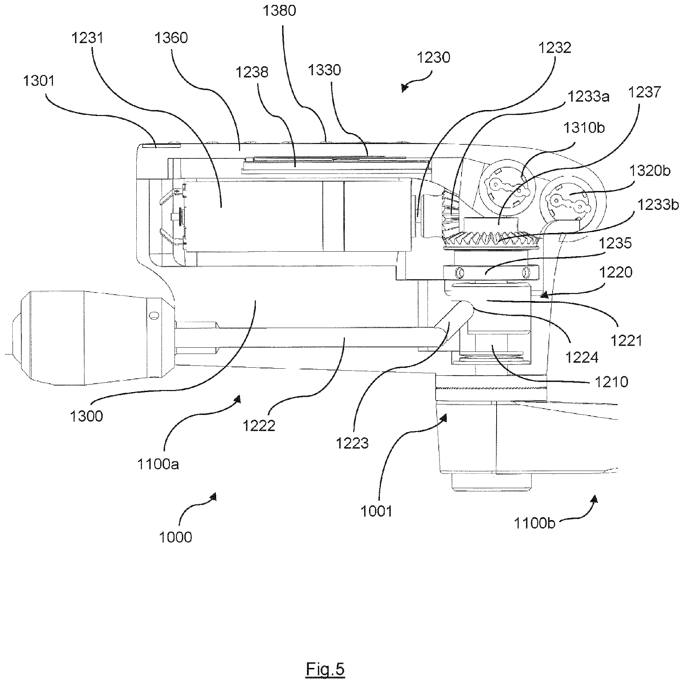

An dem Gehäuse 1300 sind Bedienelemente 1310a, 1320a zum Bedienen des Blocl<iermechanismus 1200 angeordnet. In einer Zusammenschau der Fig. 1 und der Fig. 5 ist erkennbar, dass die Bedienelemente 1310a und 1320a jeweils redundante Bedienelemente 1310b, 1320b auf der gegenüberliegenden Seite aufweisen. Hierdurch wird eine besonders sichere Bedienung ermöglicht. Dabei gehören die Bedienelemente 1310a und 1310b und die Bedienelemente 1320a und 1320b zusammen, d.h. müssen gemeinsam betätigt werden. Hier sind die Bedienelemente 1310a, 1310b, 1320a, 1320b als Knöpfe ausgebildet, die innerhalb einer Tasche 1370 in dem Gehäuse 1300, genauer dem Deckelelement 1360 hiervon, angeordnet sind. Durch die Tasche 1370 sind die Bedienelemente 1310a, 1310b, 1320a, 1320b einerseits gut taktil wahrnehmbar und andererseits vor ungewollter Bedienung geschützt.

Das gesamte Gehäuse 1300 ist ergonomisch als Griff ausgebildet. So kann ein Anwender das griffförmige Gehäuse 1300 greifen, wobei beispielsweise Daumen und Zeigefinger oder Daumen und Mittelfinger die Bedienelemente 1320a, 1320b oder 1310a, 1310b simultan betätigen können. Beispielsweise kann eine Betätigung der Bedienelemente 1310a, 1310b zu einem Blockieren der Gelenke 1001, 1110a, 1110b und eine Betätigung der Bedienelemente 1320a, 1320b zu einem Freigeben der Gelenke 1001, 1110a, 1110b führen, oder umgekehrt. Der Rest der Hand kann dann das Gehäuse 1300 greifen bzw. festhalten und das Armelement 1100a positionieren. Dabei liegt die Handfläche auf dem Gehäuse 1300 auf. Das Gehäuse 1300 kann eine Strukturierung 1380 aufweisen, die ein Greifen des Gehäuses 1300 unterstützt. Alternativ oder zusätzlich kann das Gehäuse 1300 mit einer Anti-Rutsch-Beschichtung beschichtet sein.

Das Gehäuse 1300 weist mindestens ein Indikatorelement 1340 auf, das beispielsweise dazu ausgebildet ist, einen Zustand des Blocl<iermechanismus 1200 anzuzeigen. In der Fig. 1 ist das Indikatorelement 1340 als visueller Indikator, genauer als LED, ausgebildet.

Die Fig. 2 zeigt den in der Fig. 1 dargestellten motorisierte Positionierarm 1000 ohne das Deckelement 1360. Die meisten der in der Fig. 2 dargestellten Bauteile sind bereits in Bezug auf Fig. 1 beschrieben worden. Auf eine erneute Beschreibung der bereits bekannten Aspekte wird im Folgenden größtenteils verzichtet. Sämtliche bereits in Bezug auf Fig. 1 beschriebenen Aspekte bzw. Bauteile sind allerdings auf die Fig. 2 und die folgenden Figuren übertragbar.

Da in der Fig. 2 das Deckelelement 1360 nicht dargestellt ist, sind erste Bauteile einer zu dem Blocl<iermechanismus 1200 gehörenden elektrischen Blocl<iereinrichtung 1230 zu erkennen, die in dem Gehäuse 1300 aufgenommen sind.

Dabei ist insbesondere ein Akkumulator 1238 erkennbar, der zum Antreiben der elektrischen Blocl<iereinrichtung 1230 dient. Der Akkumulator 1238 ist mittels einer Ladespule 1330 induktiv aufladbar. Die Ladespule 1330 ist zumindest im Wesentlichen direkt unter dem Deckelelement 1360 angeordnet.

Zum Aufladen des Akkumulators 1238 weist der motorisierte Positionierarm 1000 eine Ladeeinrichtung 1350 auf. Die Ladeeinrichtung 1350 ist in der Fig. 1 in einer den Akkumulator 1238 ladenden Position gezeigt, in der die Ladeeinrichtung 1350 an einer vordefinierten Position an dem Gehäuse 1300, genauer dem Deckelement 1360 hiervon, angeordnet ist. Bei der in der Fig. 2 gezeigten abgehobenen und um 90° gedrehten Ladeeinrichtung 1350 ist die zu der Ladespule 1330 korrespondierende Ladespule 1351 dargestellt.

Das Indikatorelement 1340 kann beispielsweise dazu ausgebildet sein, einen Ladezustand des Akkumulators 1238 und/oder einen Blocl<ierzustand des Blocl<iermechanismus 1200 anzuzeigen.

Von dem Blocl<iermechanismus 1200 ist in der Fig. 2 ferner eine Abdeckung 1239 der elektrischen Blocl<iereinrichtung 1230 gezeigt.

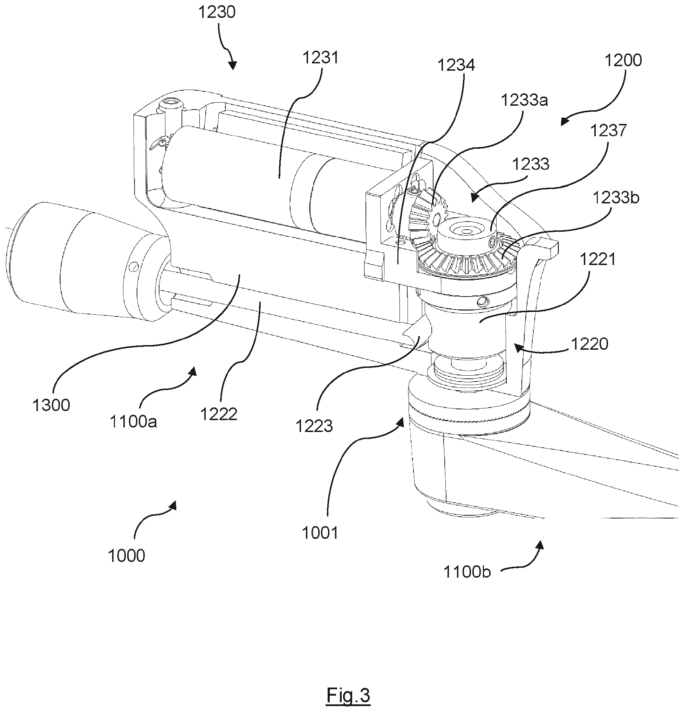

Die Fig. 3 zeigt eine etwas detailliertere Darstellung des erfindungsgemäßen motorisierten Positionierarms 1000, bei dem das Gehäuse 1300 halb ausgeblendet ist, um den Blocl<iermechanismus 1200 genauer zu zeigen.

Der Blocl<iermechanismus 1200 weist neben der elektrischen Blocl<iereinrichtung 1230 eine Übertragungseinrichtung 1220 auf, die dazu ausgebildet ist, eine Blocl<ierbewegung bzw. Freigabebewegung an die weiteren Gelenke 1110a und 1110b weiterzugeben. Die Übertragungseinrichtung 1220 ist in Fig. 3 der Einfachheit halber nur für das Armelement 1100a dargestellt.

Die Übertragungseinrichtung 1220 weist eine Spannhülse 1221 auf, die mittels eines Nockens 1223 eine Bewegung auf eine Schubstange 1222 übertragen kann. Detaillierter beschrieben, bewegt sich die Schubstange 1222 in der Fig. 3 nach links, wenn sich die Spannhülse 1221 nach unten bewegt. Durch die Bewegung der Schubstange 1222 nach links, wird das weitere Gelenk 1 1 10a blockiert.

Die elektrische Blocl<iereinrichtung 1230 weist einen Elektromotor 1231 auf. Wie in der Fig. 3 zu erkennen, ist der Elektromotor 1231 zumindest im Wesentlichen parallel zu der Axialrichtung des Armelements 1100a ausgerichtet und angeordnet. So kann der Elektromotor 1231 besonders platzsparend an dem Armelement 1 100a angeordnet werden. Durch eine derartige Anordnung des Elektromotors 1231 muss allerdings die Ausgangsbewegung des Elektromotors 1231 umgewandelt werden. Hierzu weist die elektrische Blocl<iereinrichtung 1230 ein I<egelradgetriebe 1233 auf.

Das I<egelradgetriebe 1233 ist beispielsweise in der Fig. 2 nicht sichtbar, da es durch die Abdeckung 1239 verdeckt ist. Die Abdeckung 1239 dient zum Schutz des I<egelradgetriebes 1233. In der Fig. 3 ist die Abdeckung 1239 allerdings zur besseren Erkennbarkeit des Mechanismus nicht dargestellt.

Das I<egelradgetriebe 1233 weist ein als I<egelrad ausgebildetes Eingangszahnrad 1233a und ein als I<egelrad ausgebildetes Ausgangszahnrad 1233b auf. Das Ausgangszahnrad 1233b ist größer als das Eingangszahnrad 1233a, so dass eine Übersetzung ins Langsame erfolgt, also ein Untersetzungsgetriebe vorliegt. Die Wellen, auf denen das Eingangszahnrad 1233a und das Ausgangszahnrad 1233b angeordnet sind, stehen senkrecht zueinander.

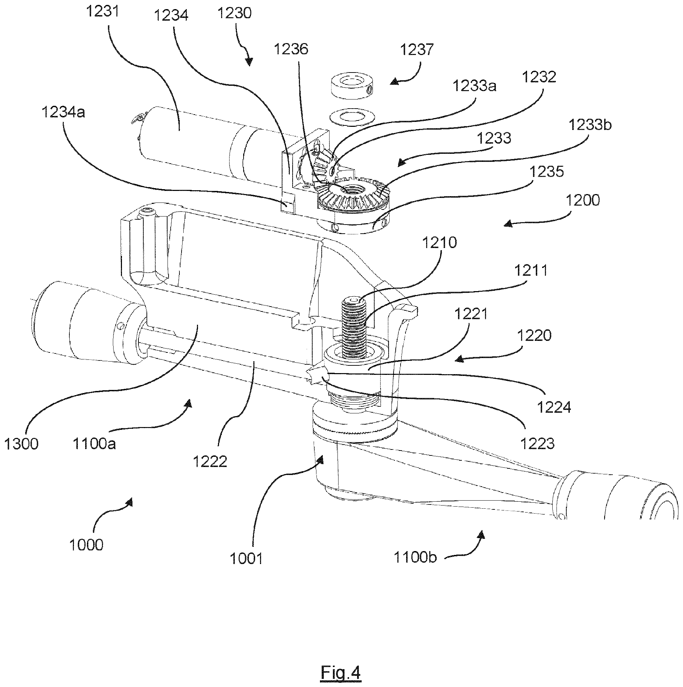

Dies ist besser in Fig. 4 zu erkennen, die eine detailliertere Darstellung des erfindungsgemäßen motorisierten Positionierarms 1000 mit einem teilweise ausgeblendeten Gehäuse 1300 und einer von dem restlichen motorisierten Positionierarm 1000 beabstandeten elektrischen Blocl<iereinrichtung 1230 zeigt. In der Fig. 4 ist die elektrische Blocl<iereinrichtung 1230von einer zentralen Welle 1210 abgehoben dargestellt. Die zentrale Welle 1210 steht mit dem Ausgangszahnrad 1233b in Wirkverbindung. Hierzu weist die zentrale Welle 1210 ein Außengewinde 1211 auf und das Ausgangszahnrad 1233b ein Innengewinde 1236 auf. Wie in der Fig. 4 zu erkennen, weist der Elektromotor 1231 eine Ausgangswelle 1232 auf, die die senkrecht zu der zentralen Welle 1210 angeordnete Welle ist, an der das Eingangszahnrad 1233a angebracht ist.

Wie in der Fig. 3 und der Fig. 4 erkennbar, weist die elektrische Blocl<iereinrichtung 1230 ein Winkelstück 1234 auf, das das I<egelradgetriebe 1233 trägt. Das Winkelstück 1234 ist ferner dazu ausgebildet, ein Mitdrehen der elektrischen Blocl<iereinrichtung 1230 im Betrieb zu verhindern. Wie in der Fig. 4 gezeigt, weist das Winkelstück 1234 hierzu einen Anschlag 1234a auf. Der Anschlag 1234a kommt mit dem Gehäuse 1300 in Kontakt und verhindert so ein Mitdrehen der elektrischen Blocl<iereinrichtung 1230.

Im Betrieb, wenn die elektrische Blocl<iereinrichtung 1230 wie in der Fig. 3 oder der Fig. 5 gezeigt an der zentralen Welle 1210 angeordnet ist, bewegt sich die elektrische Blocl<iereinrichtung 1230 entlang der zentralen Welle 1210 nach unten bzw. oben. Daher ist die elektrische Blocl<iereinrichtung 1230 schwimmend d.h. bewegbar innerhalb des Gehäuses 1300 angeordnet. Die Bewegung der elektrischen Blocl<iereinrichtung 1230 kann beispielsweise nur mittels der zentralen Welle 1210 sowie lateral durch das Gehäuse 1300 und den Anschlag 1234a geführt sein, die ein Mitdrehen verhindern. Allerdings kann in dem Gehäuse 1300 alternativ auch eine Linearführung angeordnet sein.

Beim Bewegen der elektrischen Blocl<iereinrichtung 1230 in Axialrichtung der zentralen Welle 1210 nach unten, also in Richtung der Armelemente 1100a und 1100b, werden die Gelenke 1001, 1110a und 1110b blockiert. Das Blockieren der Gelenke 1001, 1110a und 1110b muss nicht gleichzeitig erfolgen. Vielmehr wird bevorzugt, wenn die Gelenke 1001, 1110a und 1110b progressiv blockieren. Wenn die Gelenke 1001, 1110a und 1110b progressiv bzw. nacheinander blockiert bzw. freigegeben werden können, werden die Freiheitsgrade der Bewegung des motorisierten Positionierarms 1000 auch nur nach und nach beschränkt und freigegeben. So kann einerseits eine einfachere Einstellung der Position des motorisierten Positionierarms 1000 und andererseits eine gewisse Stabilisierung beim Freigeben erfolgt. Beispielsweise kann erst das weitere Gelenk 1110a, dann das zentrale Gelenk 1001 und dann das weitere Gelenk 1 1 10b blockiert werden.

In welcher Reihenfolge die Gelenke 1001, 1110a und 1110b blockieren, kann durch Anpassen der Bauteile der Übertragungseinrichtung 1200 erfolgen. Ein Anpassen kann für das Armelement 1100a durch Variation der Längen der Spannhülse 1221 und der Schubstange 1222 erfolgen. Entsprechendes gilt für das Armelement 1 100b, bei dem auch die Längen der zugehörigen Spannhülse und der zugehörigen Schubstange variiert werden können. Kürzt man beispielsweise die Spannhülse 1221, so erfolgt die Verspannung des zentralen Gelenks 1001 später als die des weiteren Gelenks 1 1 10a.

Beim Blockieren drückt die elektrische Blocl<iereinrichtung 1230, die sich aufgrund der Wirkverbindung zwischen Innengewinde 1236 und dem Außengewinde 1211 in der Fig. 5 gesehen nach unten bewegt, die Spannhülse 1221 ebenfalls nach unten. Hierdurch bewegt sich der in einer Gelenkpfanne 1224 an der Spannhülse 1221 angeordnete Nocken 1223 mit nach unten und drückt die Schubstange 1222 nach links, also in Richtung des weiteren Gelenks 1110a. Hierdurch kann das weitere Gelenk 1110a blockiert werden. Die Übertragungseinrichtung des anderen Armelements 1 100b ist nicht dargestellt. Diese kann allerdings auch mittels Spannhülse und Schubstange ausgebildet sein. Die Übertragungseinrichtung bzw. der Teil der Übertragungseinrichtung des anderen Armelements 1100b ist ähnlich aufgebaut. Beispielsweise kann eine Spannhülse des anderen Armelements 1100b allerdings ein Innengewinde aufweisen, so dass sich die Spannhülse beim Blockieren der elektrischen Blocl<iereinrichtung 1230 nach oben (Blocl<ierbewegung) bzw. nach unten (Freigabebewegung) bewegen kann.

Beim Freigeben bewegt sich die elektrische Blocl<iereinrichtung 1230 an der zentralen Welle 1210 nach oben. Um die Bewegung der elektrische Blocl<iereinrichtung 1230 nach oben zu beschränken, weist die elektrische Blocl<iereinrichtung 1230 einen Stopper 1237 auf. Der Stopper 1237 ist, wie insbesondere in der Fig. 4 gut zu erkennen, ringförmig ausgebildet und auf einem oberen Ende der zentralen Welle 1210 angeordnet.

Um die Bewegung der elektrischen Blocl<iereinrichtung 1230 zu ermöglichen, ist das Ausgangszahnrad 1233b mittels Trocl<enlager 1235 gelagert. Wie ebenfalls insbesondere in der Fig. 4 zu erkennen, sind die Trocl<enlager 1235 zumindest im Wesentlichen ringförmig ausgebildet. Beispielsweise sind diese als Gleitl<unststoffringe ausgebildet.

Wie in Fig. 5 zu erkennen, weist das Gehäuse 1300 eine Zentrierung 1301 auf. Diese Zentrierung 1301 dient dazu, ein zuverlässiges Anordnen der Ladeeinrichtung 1350 zu ermöglichen (siehe Fig.1).

## Patentansprüche

1. Motorisierter Positionierarm (1000) zum Positionieren von Instrumenten, insbesondere medizintechnischen Instrumenten, wobei der motorisierte Positionierarm (1000) mindestens zwei Armelemente (1100a, 1100b) aufweist, die mittels eines zentralen Gelenks (1001) schwenkbar um eine Schwenkachse miteinander verbunden sind,
wobei zumindest eines der Armelemente (1100a, 1100b) ein weiteres Gelenk (1110a, 1110b) an einem dem zentralen Gelenk (1001) entgegengesetzten Ende (1120a, 1120b) aufweist,
wobei der motorisierte Positionierarm (1000) einen Blocl<iermechanismus (1200) zum Blockieren und Freigeben des zentralen Gelenks (1001) und des zumindest einen weiteren Gelenks (1110a, 1 1 10b) aufweist, und
wobei der Blocl<iermechanismus (1200) eine zentrale Welle (1210), die koaxial zu der Schwenkachse angeordnet ist, und zumindest eine Übertragungseinrichtung (1220) von der zentralen Welle (1210) zu dem zumindest einen weiteren Gelenk (1110a, 1110b) aufweist,
**dadurch gekennzeichnet, dass** der Blocl<iermechanismus (1200) eine elektrische Blocl<iereinrichtung (1230) aufweist, die dazu ausgebildet ist, mit der zentralen Welle (1210) in Eingriff zu stehen, um das Blockieren und Freigeben der Gelenke (1001, 1110a, 1110b) zu ermöglichen,
wobei die elektrische Blocl<iereinrichtung (1230) dazu ausgebildet ist, sich entlang der zentralen Welle (1210) zu bewegen.

2. Motorisierter Positionierarm (1000) nach Anspruch 1,
wobei beide Armelemente (1100a, 1100b) jeweils das weitere Gelenk (1110a, 1110b) an dem dem zentralen Gelenk (1001) entgegengesetzten Ende (1120a, 1120b) aufweisen, und
wobei der Blocl<iermechanismus (1200) jeweils eine Übertragungseinrichtung (1220) zu dem weiteren Gelenk (1110a, 1110b) aufweist.

3. Motorisierter Positionierarm (1000) nach Anspruch 1 oder 2,
wobei das weitere Gelenk (1110a, 1110b) als I<ugelgelenl< ausgebildet ist.

4. Motorisierter Positionierarm (1000) nach einem der Ansprüche 1 bis 3,
wobei die elektrische Blocl<iereinrichtung (1230) einen Elektromotor (1231) mit einer Ausgangswelle (1232) aufweist, wobei die Ausgangswelle (1232) senkrecht zu der zentralen Welle (1210) ausgerichtet ist.

5. Motorisierter Positionierarm (1000) nach Anspruch 4,
wobei die elektrische Blocl<iereinrichtung (1230) ein I<egelradgetriebe (1233) mit einem Eingangszahnrad (1233a), das mit der Ausgangswelle (1232) des Elektromotors (1231) verbunden ist, und einem Ausgangszahnrad (1233b) aufweist, das mit der zentralen Welle (1210) in Eingriff ist.

6. Motorisierter Positionierarm (1000) nach Anspruch 5
wobei das Ausgangszahnrad (1233b) mittels zumindest im Wesentlichen ringförmigen Trocl<enlager (1235) gelagert ist.

7. Motorisierter Positionierarm (1000) nach Anspruch 5 oder 6,
wobei das Ausgangszahnrad (1233b) ein Innengewinde (1236) aufweist und die zentrale Welle (1210) ein zu dem Innengewinde (1236) passendes Außengewinde (1211) aufweist.

8. Motorisierter Positionierarm (1000) nach einem der Ansprüche 4 bis 7,
wobei die elektrische Blocl<iereinrichtung (1230) an einem (1100a) der beiden Armelemente (1100a, 1100b) angeordnet ist und sich die Ausgangswelle (1232) des Elektromotors (1231) zumindest im Wesentlichen parallel zu der Axialrichtung des Armelements (1100a) erstreckt, an dem die elektrische Blocl<iereinrichtung (1230) angeordnet ist.

9. Motorisierter Positionierarm (1000) nach Anspruch 8,
wobei die elektrische Blocl<iereinrichtung (1230) lösbar an dem Armelement (1100a) ausgebildet ist, an dem die elektrische Blocl<iereinrichtung (1230) angeordnet ist.

10. Motorisierter Positionierarm (1000) nach Anspruch 8 oder 9,
wobei die elektrische Blocl<iereinrichtung (1230) als bewegliche Spanneinheit ausgebildet ist, die relativ zu dem Armelement (1100a), an dem die elektrische Blocl<iereinrichtung (1230) angeordnet ist, in Axialrichtung der zentralen Welle (1210) bewegbar ausgebildet ist.

11. Motorisierter Positionierarm (1000) nach einem der vorangehenden Ansprüche, wobei der motorisierte Positionierarm (1000) ein Gehäuse (1300) aufweist, von dem die elektrische Blocl<iereinrichtung (1230) zumindest im Wesentlichen umschließbar ist, wobei das Gehäuse (1300) griffförmig ausgebildet ist.

12. Motorisierter Positionierarm (1000) nach Anspruch 11,
wobei die elektrische Blocl<iereinrichtung (1230) einen Anschlag (1234a) aufweist, der mit dem Gehäuse (1300) in Anschlag gerät und dadurch ein Mitdrehen der elektrischen Blocl<iereinrichtung (1230) beim Blockieren und Freigeben der Gelenke (1001, 1110a, 1110b) verhindert.

13. Motorisierter Positionierarm (1000) nach Anspruch 11 oder 12,
wobei an dem Gehäuse (1300) redundante Bedienelemente (1310a, 1310b, 1320a, 1320b) zum Ansteuern des Elektromotors (1231) angeordnet sind.

14. Motorisierter Positionierarm (1000) nach einem der Ansprüche 11 bis 13,
wobei innerhalb des Gehäuses (1300) eine Ladespule (1330) zum I<abellosen Laden eines zum Antrieb des Elektromotors (1231) geeigneten Akkumulators (1238) angeordnet ist.

15. Motorisierter Positionierarm (1000) nach einem der Ansprüche 11 bis 14,
wobei an dem Gehäuse (1300) mindestens ein Indikatorelement (1340) angeordnet ist, mittels dessen ein Ladezustand des Akkumulators (1238) und/oder ein Blocl<ierzustand des Blocl<iermechanismus (1200) anzeigbar ist.

## Claims

1. Motorized positioning arm (1000) for positioning instruments, in particular medical instruments, wherein the motorized positioning arm (1000) comprises at least two arm elements (1100a, 1100b) which are connected to one another so as to be pivotable about a pivot axis by means of a central joint (1001),
wherein at least one of the arm elements (1100a, 1100b) comprises a further joint (1110a, 1110b) at an end (1120a, 1120b) opposite to the central joint (1001),
wherein said motorized positioning arm (1000) comprises a blocking mechanism (1200) for blocking and releasing the central joint (1001) and the at least one further joint (1110a, 1110b), and
wherein the blocking mechanism (1200) comprises a central shaft (1210) arranged coaxially with the pivot axis, and at least one transmission device (1220) from the central shaft (1210) to the at least one further joint (1110a, 1110b)
**characterized in that** the blocking mechanism (1200) comprises an electrical blocking device (1230) configured to engage with the central shaft (1210) to enable blocking and unblocking of the joints (1001, 1110a, 1110b),
wherein the electrical blocking device (1230) is configured to move along the central shaft (1210).

2. The motorized positioning arm (1000) according to claim 1,
wherein both arm elements (1100a, 1100b) each comprise the further joint (1110a, 1110b) at the end (1120a, 1120b) opposite the central joint (1001), and
wherein the blocking mechanism (1200) comprises a respective transmission device (1220) to the further joint (1110a, 1110b).

3. The motorized positioning arm (1000) according to claim 1 or 2,
wherein the further joint (11 10a, 11 10b) is configured as a ball joint.

4. The motorized positioning arm (1000) according to any one of claims 1 to 3,
wherein the electric blocking device (1230) comprises an electric motor (1231) with an output shaft (1232), wherein the output shaft (1232) is arranged perpendicular to the central shaft (1210).

5. The motorized positioning arm (1000) of claim 4,
wherein the electric blocking device (1230) comprises a bevel gearing (1233) with an input gear (1233a) connected to the output shaft (1232) of the electric motor (1231) and an output gear (1233b) engaged with the central shaft (1210).

6. The motorized positioning arm (1000) of claim 5.
wherein the output gear (1233b) is supported by an at least substantially annular dry bearing (1235).

7. The motorized positioning arm (1000) according to claim 5 or 6,
wherein the output gear (1233b) comprises an internal thread (1236) and the central shaft (1210) comprises an external thread (1211) complementary to the internal thread (1236).

8. The motorized positioning arm (1000) according to any one of claims 4 to 7,
wherein the electric blocking device (1230) is arranged on one (1100a) of the two arm elements (1100a, 1100b) and the output shaft (1232) of the electric motor (1231) extends at least substantially parallel to the axial direction of the arm element (1 100a) on which the electric blocking device (1230) is arranged.

9. The motorized positioning arm (1000) according to claim 8,
wherein the electrical blocking device (1230) is releasably arranged on the arm element (1100a) on which the electrical blocking device (1230) is arranged.

10. The motorized positioning arm (1000) according to claim 8 or 9,
wherein the electric blocking device (1230) is configured as a movable clamping unit, which is configured movable in axial direction of the central shaft (1210) relative to the arm element (1 100a) on which the electric blocking device (1230) is arranged.

11. The motorized positioning arm (1000) according to one of the preceding claims,
wherein the motorized positioning arm (1000) comprises a housing (1300) capable of at least substantially enclosing the electrical blocking device (1230), wherein the housing (1300) is configured in the shape of a handle.

12. The motorized positioning arm (1000) according to claim 11,
wherein the electrical blocking device (1230) comprises a stop (1234a) which comes into abutment with the housing (1300) and thereby prevents the electrical blocking device (1230) from rotating with the housing (1300) when blocking and releasing the joints (1001, 1110a, 1110b).

13. The motorized positioning arm (1000) according to claim 11 or 12,
wherein redundant operating elements (1310a, 1310b, 1320a, 1320b) are arranged on the housing (1300) for controlling the electric motor (1231).

14. The motorized positioning arm (1000) according to any one of claims 11 to 13,
wherein a charging coil (1330) is arranged within the housing (1300) for wireless charging of an accumulator (1238) suitable for driving the electric motor (1231).

15. The motorized positioning arm (1000) according to any one of claims 11 to 14,
wherein at least one indicator element (1340) is arranged on the housing (1300), by means of which a charging state of the accumulator (1238) and/or a blocking state of the blocking mechanism (1200) can be indicated.

## Revendications

1. Bras de positionnement motorisé (1000) pour le positionnement d'instruments, en particulier d'instruments de technique médicale, dans lequel le bras de positionnement motorisé (1000) présente au moins deux éléments de bras (1100a, 1100b) qui sont reliés entre eux au moyen d'une articulation centrale (1001) de manière à pouvoir pivoter autour d'un axe de pivotement,
dans lequel au moins l'un des éléments de bras (1100a, 1100b) comporte une articulation supplémentaire (1110a, 1110b) à une extrémité (1120a, 1120b) opposée à l'articulation centrale (1001),
dans lequel le bras de positionnement motorisé (1000) comprend un mécanisme de blocage (1200) pour bloquer et débloquer l'articulation centrale (1001) et l'au moins une articulation supplémentaire (1 1 10a, 1110b), et
dans lequel le mécanisme de blocage (1200) comprend un arbre central (1210) disposé de manière coaxiale par rapport à l'axe de pivotement et au moins un moyen de transmission (1220) de l'arbre central (1210) à l'au moins une articulation supplémentaire (1110a, 1110b),
**caractérisé en ce que** le mécanisme de blocage (1200) comprend un dispositif de blocage électrique (1230) adapté pour s'engager avec l'arbre central (1210) afin de permettre le blocage et le déblocage des articulations (1001, 1 1 10a, 1110b),
dans lequel le dispositif de blocage électrique (1230) est adapté pour se déplacer le long de l'arbre central (1210).

2. Bras de positionnement motorisé (1000) selon la revendication 1,
dans lequel les deux éléments de bras (1100a, 1100b) comprennent chacun l'articulation supplémentaire (1110a, 1110b) à l'extrémité (1120a, 1120b) opposée à l'articulation centrale (1001), et
dans lequel le mécanisme de blocage (1200) comprend un dispositif de transmission (1220) vers l'articulation supplémentaire (11 10a, 1110b), respectivement.

3. Bras de positionnement motorisé (1000) selon la revendication 1 ou 2,
dans lequel l'articulation supplémentaire (1 1 10a, 1 1 10b) est une articulation à rotule.

4. Bras de positionnement motorisé (1000) selon l'une des revendications 1 à 3,
dans lequel le dispositif de blocage électrique (1230) comprend un moteur électrique (1231) ayant un arbre de sortie (1232), dans lequel l'arbre de sortie (1232) est orienté perpendiculairement à l'arbre central (1210).

5. Bras de positionnement motorisé (1000) selon la revendication 4,
dans lequel le dispositif de blocage électrique (1230) comprend un engrenage conique (1233) comportant un pignon d'entrée (1233a) relié à l'arbre de sortie (1232) du moteur électrique (1231) et un pignon de sortie (1233b) en contact avec l'arbre central (1210).

6. Bras de positionnement motorisé (1000) selon la revendication 5.
dans lequel le pignon de sortie (1233b) est supporté par des paliers secs (1235) au moins essentiellement annulaires.

7. Bras de positionnement motorisé (1000) selon la revendication 5 ou 6,
dans lequel le pignon de sortie (1233b) présente un filetage intérieur (1236) et l'arbre central (1210) présente un filetage extérieur (1211) adapté au filetage intérieur (1236).

8. Bras de positionnement motorisé (1000) selon une des revendications 4 à 7,
dans lequel le dispositif de blocage électrique (1230) est disposé sur l'un (1100a) des deux éléments de bras (1100a, 1100b) et l'arbre de sortie (1232) du moteur électrique (1231) s'étend au moins essentiellement parallèlement à la direction axiale de l'élément de bras (1100a) sur lequel est disposé le dispositif de blocage électrique (1230).

9. Bras de positionnement motorisé (1000) selon la revendication 8,
dans lequel le dispositif de blocage électrique (1230) est formé de manière amovible sur l'élément de bras (1100a) sur lequel est disposé le dispositif de blocage électrique (1230).

10. Bras de positionnement motorisé (1000) selon la revendication 8 ou 9,
dans lequel le dispositif de blocage électrique (1230) est conçu comme une unité de serrage mobile qui est conçue pour se déplacer dans la direction axiale de l'arbre central (1210) par rapport à l'élément de bras (1100a) sur lequel le dispositif de blocage électrique (1230) est disposé.

11. Bras de positionnement motorisé (1000) selon l'une des revendications précédentes,
dans lequel le bras de positionnement motorisé (1000) comporte un boîtier (1300), le dispositif de blocage électrique (1230) étant configuré pour pouvoir être entouré au moins essentiellement dudit boîtier, dans lequel le boîtier (1300) est réalisé en forme de poignée.

12. Bras de positionnement motorisé (1000) selon la revendication 11,
dans lequel le dispositif de blocage électrique (1230) comporte une butée (1234a) qui vient en butée avec le boîtier (1300) et empêche ainsi l'entraînement en rotation du dispositif de blocage électrique (1230) lors du blocage et du déblocage des articulations (1001, 1110a, 1110b).

13. Bras de positionnement motorisé (1000) selon la revendication 11 ou 12,
dans lequel des éléments de commande redondants (1310a, 1310b, 1320a, 1320b) sont disposés sur le boîtier (1300) pour commander le moteur électrique (1231).

14. Bras de positionnement motorisé (1000) selon l'une des revendications 11 à 13,
dans lequel une bobine de charge (1330) est disposée à l'intérieur du boîtier (1300) pour charger sans fil un accumulateur (1238) adapté au fonctionnement du moteur électrique (1231).

15. Bras de positionnement motorisé (1000) selon l'une quelconque des revendications 11 à 14,
dans lequel au moins un élément indicateur (1340) est disposé sur le boîtier (1300), au moyen duquel un état de charge de l'accumulateur (1238) et/ou un état de blocage du mécanisme de blocage (1200) est affichable.
